# FASCICULE DE BREVET EUROPEEN

(11) **EP 0 514 303 B1**
(45) Date de publication et mention de la délivrance du brevet: **29.11.1995**
(21) Numéro de dépôt: 92450005.1
(22) Date de dépôt: 14.05.1992
(51) Int. Cl.: A61B 17/58

(54) **Dispositif de liaison verrouillable d'éléments d'ancrage d'ostéosynthèse rachidienne**
Verriegelbares Verbindungsstück für Verankerungselemente für die Wirbelsäulenosteosynthese
Lockable connecting device for spiral osteosynthesis anchoring elements

(30) Priorité: 17.05.1991 FR 9106133
(43) Date de publication de la demande: 19.11.1992
(73) Titulaire: STRYKER CORPORATION (a Michigan corporation), Kalamazoo, Michigan 49002 (US); MISSENARD, Gilles, F-75016 Paris (FR); LAPRESLE, Philippe, F-92200 Neuilly-sur-Seine (FR)
(72) Inventeur: Vignaud, Jean-Louis, F-33950 Le Piraillan - Lege - Cap Ferret (FR); Missenard, Gilles, F-75016Paris (FR); Lapresle, Philippe, F-92200 Neuilly-sur-Seine (FR); Sacriste, Jean-François, F-33115 Le Pyla-sur-Mer (FR)
(74) Mandataire: Martin, Jean-Jacques

(56) Documents cités:
- EP-A- 0 348 272
- EP-A- 0 350 925
- EP-A- 0 383 992
- DE-A- 3 807 335

## Description

La présente invention a trait à un dispositif permettant la liaison et le blocage en position de deux éléments d'ancrage d'ostéosynthese rachidienne.

L'invention concerne plus particulièrement mais non exclusivement les dispositifs d'ostéosynthèse rachidienne du type comprenant une tige reçue et bloquée dans un certain nombre d'éléments d'ancrage constitués d'une partie d'ancrage osseux formée par une vis ou une lame recourbée et d'un corps de fixation de la tige constitué d'une tête dite en forme de diapason recevant entre ses deux branches ladite tige qui est bloquée à l'aide d'une vis-pointeau reçue dans un taraudage ménagé sur les faces internes en regard desdites branches.

Habituellement, par exemple en cas de fracture d'une vertèbre du rachis, deux tiges d'ostéosynthèse sont fixées en parallèle de part et d'autre du rachis à l'aide d'éléments d'ancrage fixés sur certaines des vertèbres.

Ce dispositif, tel que décrit par exemple dans le document EP-A-0 348 272, est complété par des dispositifs de solidarisation et de traction transversales reliant les deux tiges, constitués généralement par des tiges transversales reliant des crochets engagés sur les deux tiges.

Ces dispositifs ne sont pas pratiques à mettre en place et sont de nature à tordre les tiges du fait des tractions qu'ils peuvent exercer sur ces dernières.

Le but de l'invention est de proposer notamment un dispositif de solidarisation en traction transversale de tels dispositifs d'ostéosynthèse plus simple de conception, plus facile à mettre en place et plus efficace.

A cet effet, l'invention a pour objet un dispositif de liaison verrouillable de deux éléments d'ancrage d'ostéosynthèse rachidienne, lesdits éléments d'ancrage comprenant une partie de fixation osseuse formée d'une vis, d'une lame recourbée ou analogue et un corps de fixation d'une tige de solidarisation formé d'une tête en diapason recevant ladite tige ainsi qu'une vis de blocage, caractérisé en ce qu'il est constitué de deux éléments annulaires susceptibles d'être enfilés chacun extérieurement sur l'une des têtes en diapason, chaque élément annulaire comprenant une tige externe de blocage pouvant coulisser dans un collier de serrage, les deux colliers de serrage du dispositif étant serrés par une vis commune traversant lesdits colliers.

Chaque élément annulaire enfilé sur une tête en diapason est bloqué sur celle-ci lors du vissage de la vis de blocage de la tige de liaison des éléments d'ancrage, cette opération ayant pour effet d'écarter les branches du diapason et de verrouiller ainsi l'élément annulaire entourant la tête en diapason.

Ensuite, la tige externe de chaque élément annulaire est ajustée par coulissement à l'intérieur de son collier puis, la position correcte étant trouvée pour les deux tiges externes, le simple vissage de la vis unique bloque les deux colliers de serrage.

Les efforts de traction du dispositif de solidarisation sont ainsi exercés directement sur les éléments d'encrage et non plus sur les tiges de liaison, entre deux éléments d'ancrage et les divers degrés de liberté des tiges de blocage des éléments annulaires ainsi que des colliers de serrage autorisent une grande souplesse dans le positionnement des éléments annulaires sur les têtes en diapason, quelle que soit leur position ou orientation éventuelle.

L'invention s'applique également à la liaison entre deux éléments d'ancrage disposés d'un même côté du rachis et appartenant à deux dispositifs d'ostéosynthèse du type ci-dessus et disposés sensiblement dans le prolongement l'un de l'autre, le dispositif de l'invention étant monté entre les deux éléments d'ancrage d'extrémité.

D'autres caractéristiques et avantages ressortiront de la description qui va suivre d'un mode de réalisation du dispositif de l'invention, description donnée à titre d'exemple uniquement et en regard des dessins annexés sur lesquels :
- Figure 1 illustre une implantation sur un rachis d'un dispositif d'ostéosynthèse rachidienne de type connu;
- Figure 2 représente en perspective une vis pédiculaire à tête en diapason de type connu ;
- Figure 3 est une vue en perspective et sensiblement en élévation d'un dispositif conforme à l'invention ;
- Figure 4 est une vue de dessus du dispositif de la figure 3, et
- Figure 5 est une vue en coupe axiale partielle d'une vis du type de la figure 2 sur laquelle est enfilé un élément annulaire du dispositif des figures 3 et 4.

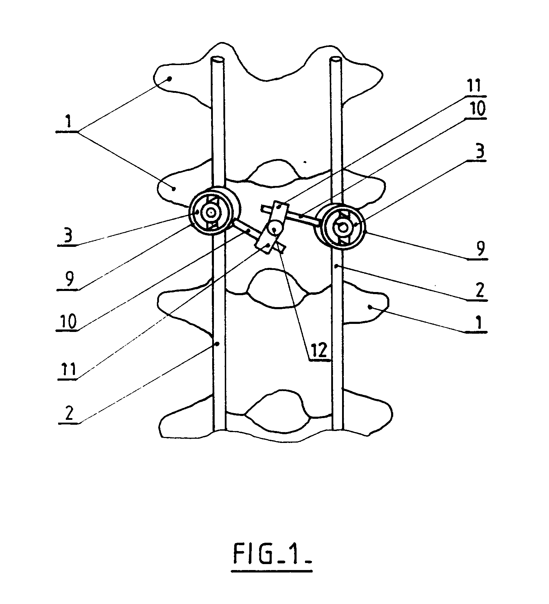

On a représenté en 1 sur la figure 1 des vertèbres munies d'une instrumentation rachidienne comprenant, de part et d'autre du rachis, une tige 2 de solidarisation reliant un certain nombre d'éléments d'ancrage constitués d'une partie de fixation osseuse, formée par une vis ou une lame recourbée par exemple, et d'une tête de réception et blocage de ladite tige 2.

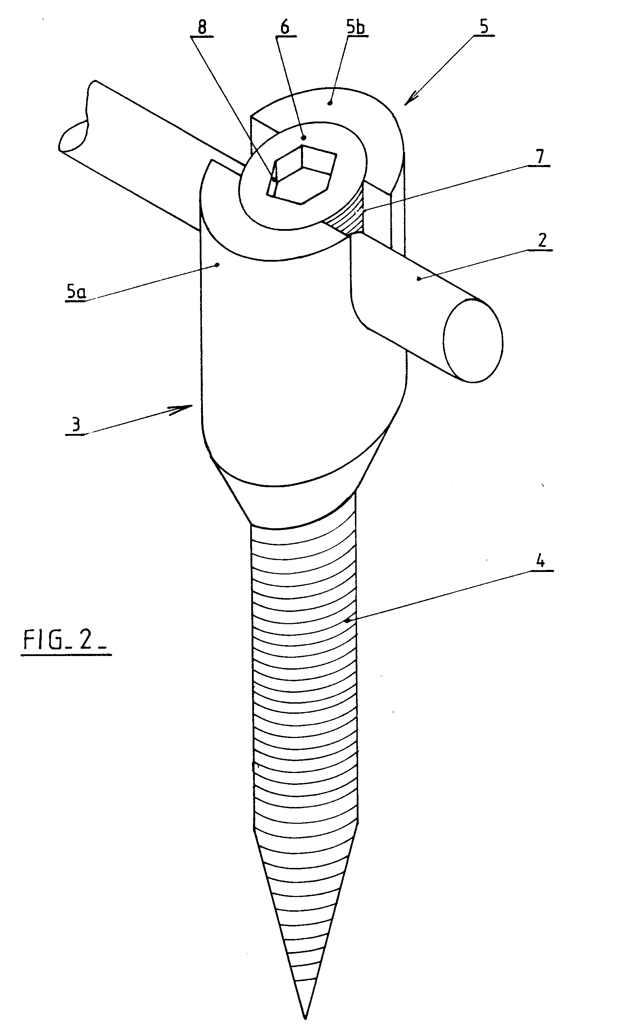

Sur la figure 1, on a représenté pour chaque tige 2 un seul de ces éléments d'ancrage, par exemple une vis pédiculaire 3 de type connue dont une vue plus détaillée est donnée en figure 2.

Une telle vis 3 comprend une tige filetée 4 d'ancrage osseux, une tête cylindrique 5 en forme de diapason entre les deux branches 5a et 5b de laquelle la tige 2 est maintenue bloquée par une vis-pointeau 6 engagée entre les branches 5a, 5b et vissée dans un taraudage 7 ménagé sur les faces internes en regard des dites branches. La vis de blocage 6 est munie sur sa face supérieure d'une empreinte en creux polygonale pour la réception d'une clé appropriée de blocage de la tige 2.

Un tel dispositif est bien connu et est généralement complété par un dispositif de solidarisation et de traction transversale reliant les deux tiges 2 sensiblement parallèles.

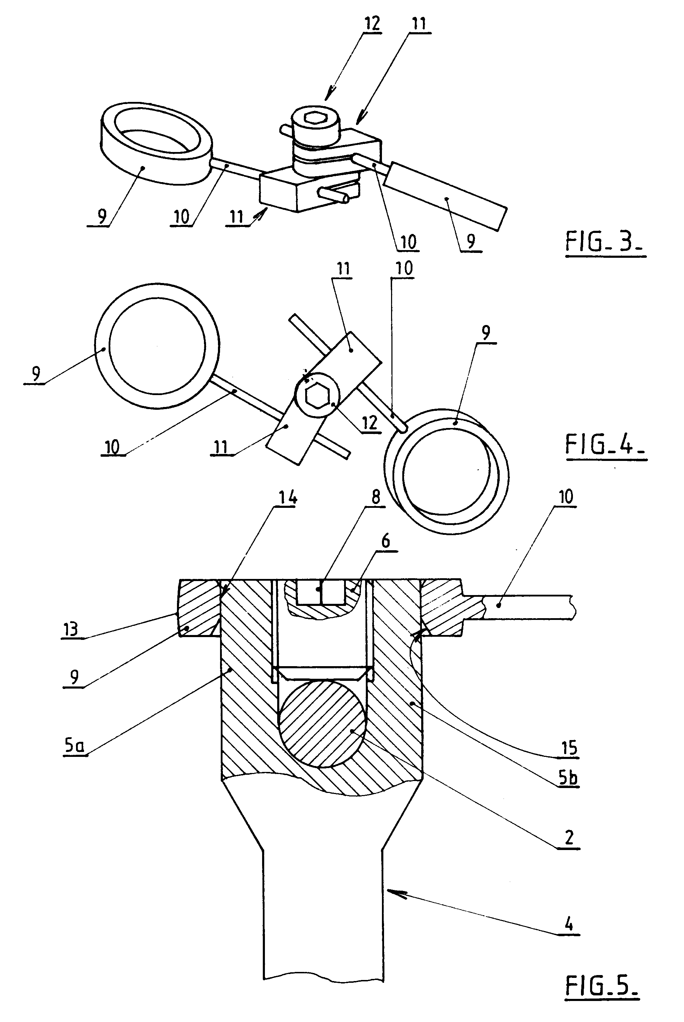

Conformément à l'invention, un tel dispositif de solidarisation et traction transversale est constitué, comme schématisé sur la figure 1 et représenté plus en détail, suivant un mode de réalisation particulier, sur les figures 3 et 4.

Le dispositif de l'invention comprend deux éléments annulaires 9 munis chacun extérieurement d'une tige de blocage 10 d'une certaine longueur. L'axe de la tige 10 passe par exemple par le centre de l'élément auxiliaire 9 mais pourrait avoir une orientation quelconque dans le plan de l'élément annulaire aussi bien qu'une inclinaison quelconque par rapport à ce plan.

Chaque tige de blocage 10 peut coulisser et être bloquée dans un collier de serrage 11.

Dans le mode de réalisation représenté les deux colliers de serrage 11 sont superposés et traversés par une vis commune 12 de serrage, orthogonale aux deux tiges 10. La vis est, par exemple, une vis à tête à six pans creux comme la vis 6.

Chaque élément annulaire 9 peut comporter, comme représenté en figure 5, une face externe convexe 13, une face interne cylindrique 14 dont le diamètre correspond au diamètre externe des têtes 5 des vis pédiculaires ou autre éléments d'ancrage similaires 3 et, du côté destiné à être présenté sur les têtes des éléments d'ancrage (3), d'un chanfrein 15 sur l'arête interne facilitant la mise en place.

Chaque élément annulaire 9 est enfilé sur une tête 5, avant serrage de la tige 2 par la vis 6, puis bloqué en place par serrage de ladite vis 6, lequel tend à écarter les branches 5a, 5b et donc à verrouiller ledit élément 9.

Les deux éléments 9 sont mis en place entre deux éléments d'ancrage 3 en regard comme illustré par la figure 1.

Ces deux éléments 9 peuvent être également mis en place sur deux éléments d'ancrage disposés du même côté du rachis. Dans ce cas, chaque élément d'ancrage 3 est, par exemple, une vis pédiculaire de fixation de l'extrémité d'une tige de liaison, le dispositif de l'invention réalisant ainsi un pontage entre les deux dispositifs d'ostéosynthèse agencés sensiblement dans le prolongement l'un de l'autre.

La mise en place des éléments 9 sur les têtes 5 est aisée, quelles que soient les positions ou orientations éventuelles desdites têtes 5, grâce aux divers degrés de liberté des organes de liaison entre les deux éléments 9. En effet, chaque tige 10 peut coulisser dans son collier de serrage 11 et pivoter autour de son axe. De plus, les deux colliers 11 peuvent pivoter angulairement l'un par rapport à l'autre de 360° autour de l'axe commun de la vis 12.

Une fois le placement correct des tiges 10 et colliers 11 obtenu, une simple rotation de l'unique vis 12 bloque l'ensemble en position.

Enfin, l'invention n'est évidemment pas limitée au mode de réalisation représenté et décrit ci-dessus mais en couvre au contraire toutes les variantes notamment en ce qui concerne l'agencement et le disposition éventuelle des colliers de serrage 11.

C'est ainsi qu'au lieu d'un plan de contact entre les deux colliers de serrage 11 perpendiculaire à l'axe de la vis 12, comme représenté sur les figures 3 et 4, on pourrait prévoir un plan de contact faisant avec l'axe de la vis 12 un angle différent de l'angle droit ce qui donnerait des possibilités supplémentaires de positionnement des éléments annulaires 9.

Les éléments du dispositif de l'invention sont, bien entendu, dans un matériau biocompatible homologué pour l'implantation chirurgicale.

## Revendications

1. Dispositif de liaison verrouillable de deux éléments d'ancrage (3) d'ostéosynthèse rachidienne, lesdits éléments d'ancrage (3) comprenant une partie de fixation osseuse (4) formée d'une vis, d'une lame recourbée ou analogue et un corps de fixation d'une tige de solidarisation formé d'une tête en diapason (5) recevant ladite tige, ainsi qu'une vis de blocage (6), caractérisé en ce qu'il est constitué de deux éléments annulaires (9) susceptibles d'être enfilés chacun extérieurement sur l'une des têtes en diapason (5), chaque élément annulaire (9) comprenant une tige externe de blocage (10) pouvant coulisser dans un collier de serrage (11), les deux colliers de serrage (11) du dispositif étant serrés par une vis commune (12) traversant lesdits colliers.

2. Dispositif suivant la revendication 1, caractérisé en ce que les deux colliers de serrage (11) sont au moins partiellement superposés et libres en rotation autour de l'axe de ladite vis commune de serrage (12) en sorte de permettre auxdites tiges de blocages (10) de se déplacer dans deux plans parallèles, perpendiculaires à l'axe de ladite vis (12).

3. Dispositif suivant la revendication 1, caractérisé en ce que les deux colliers de serrage (11) sont au moins partiellement superposés et peuvent pivoter l'un sur l'autre et par rapport à l'autre suivant un plan de contact formant avec l'axe de la vis commune de serrage (12) un angle différent de 90°.

4. Dispositif suivant l'une des revendications 1 à 3, caractérisé en ce que chaque élément annulaire (9) comporte une face interne cylindrique (14) et un des bords internes chanfreiné (15).

5. Dispositif suivant l'une des revendications 1 à 4, caractérisé en ce qu'au moins l'un des éléments annulaires (9) comporte une tige externe (10) dont l'axe passe par le centre dudit élément annulaire.

## Claims

1. Device for lockable connection of two anchoring elements (3) for spinal osteosynthesis, the said anchoring elements (3) comprising a bone fixation part (4) formed by a screw, by a curved blade or the like, and a body for securing a connecting rod, the said body being formed by a U-shaped head (5) which receives the said rod, and also a blocking screw (6), characterized in that it consists of two annular elements (9) which are capable of being engaged in each case externally on one of the U-shaped heads (5), each annular element (9) comprising an external blocking rod (10) which can slide in a clamping collar (11), the two clamping collars (11) of the device being clamped by a common screw (12) passing through the said collars.

2. Device according to Claim 1, characterized in that the two clamping collars (11) are at least partially superposed and free in terms of rotation about the axis of the said common clamping screw (12) so as to allow the said blocking rods (10) to move in two parallel planes perpendicular to the axis of the said screw (12).

3. Device according to Claim 1, characterized in that the two clamping collars (11) are at least partially superposed and can pivot on one another and with respect to one another in a plane of contact which forms, with the axis of the common clamping screw (12), an angle other than 90°.

4. Device according to one of Claims 1 to 3, characterized in that each annular element (9) has a cylindrical inner face (14) and one of the inner edges bevelled (15).

5. Device according to one of Claims 1 to 4, characterized in that at least one of the annular elements (9) comprises an external rod (10) whose axis passes through the centre of the said annular element.

## Patentansprüche

1. Verriegelbares Verbindungsstück für zwei Verankerungselemente (3) für die Wirbelsäulenosteosynthese, wobei die Verankerungselemente (3) ein Teil (4) für die Knochenfixierung, gebildet aus einer Schraube, einem gekrümmten Streifen oder dergleichen und einem Körper für die Fixierung einer Verstärkungsstange, gebildet aus einem gegabelten Kopf (5), der die Stange aufnimmt, ebenso wie eine Blockierschraube (6) aufweisen, dadurch gekennzeichnet, daß es aus zwei ringförmigen Elemente (9) gebildet ist, die dazu geeignet sind, jedes außen auf einen der gegabelten Köpfe (5) aufgeschraubt zu werden, wobei jedes ringförmige Element (9) eine äußere Blockierstange (10) aufweist, die in einem Klemmflansch (11) gleiten kann, wobei die beiden Klemmflansche (11) des Stückes durch eine gemeinsame Schraube (12) verklemmt sind, die die Flansche durchquert.

2. Stück nach Anspruch 1, dadurch gekennzeichnet, daß die beiden Klemmflansche (11) wenigstens teilweise übereinandergelegt sind und sich frei um die Achse der gemeinsamen Klemmschraube (12) drehen können, derart, daß es den Blockierstangen (10) ermöglicht wird, sich in zwei parallelen, senkrecht zu der Achse der Schraube (12) liegenden Ebenen zu verlagern.

3. Stück nach Anspruch 1, dadurch gekennzeichnet, daß die beiden Klemmflansche (11) wenigstens teilweise übereinandergelegt sind und sich der eine auf dem anderen und in bezug auf den anderen entlang einer Kontaktebene, die mit der Achse der gemeinsamen Klemmschrauben (12) einen von 90° unterschiedlichen Winkel bildet, verschwenken kann.

4. Stück nach einem der Ansprüche 1 bis 3, dadurch gekennzeichnet, daß jedes ringförmige Element (9) eine innere zylindrische Fläche (14) aufweist und einer der Innenkanten angefast (15) ist.

5. Stück nach einem der Ansprüche 1 bis 4, dadurch gekennzeichnet, daß wenigstens eines der ringförmigen Elemente (9) eine äußere Stange (10) aufweist, deren Achse durch den Mittelpunkt des ringförmigen Elementes läuft.
